# EUROPEAN PATENT SPECIFICATION

(11) **EP 3 331 443 B1**
(45) Date of publication and mention of the grant of the patent: **30.09.2020**
(21) Application number: 16751115.3
(22) Date of filing: 04.08.2016
(51) Int. Cl.: A61B 5/15, A61B 5/151

(54) **BIOLOGICAL FLUID COLLECTION DEVICE**
VORRICHTUNG ZUM SAMMELN EINES BIOLOGISCHEN FLUIDS
DISPOSITIF DE COLLECTE DE FLUIDE BIOLOGIQUE

(30) Priority: 06.08.2015 US 201562201776 P
(43) Date of publication of application: 13.06.2018
(73) Proprietor: Becton, Dickinson and Company, Franklin Lakes, NJ 07417 (US)
(72) Inventor: WILKINSON, Bradley, M., North Haledon, New Jersey 07508 (US)
(74) Representative: dompatent von Kreisler Selting Werner - Partnerschaft von Patent- und Rechtsanwälten mbB
(86) International application number: PCT/US2016/045515
(87) International publication number: WO 2017/024117

(56) References cited:
- WO-A1-99/56622
- WO-A1-2015/020609
- DE-U1- 8 513 365
- US-A- 4 920 977

## Description

### BACKGROUND OF THE DISCLOSURE

### Field of the Disclosure

The present disclosure relates generally to a biological fluid collection device. More particularly, the present disclosure relates to a biological fluid collection device having an integral collection tube and lancing device.

### Description of the Related Art

Blood sampling is a common health care procedure involving the withdrawal of at least a drop of blood from a patient. Blood samples are commonly taken from hospitalized, homecare, and emergency room patients either by finger stick, heel stick, or venipuncture. Blood samples may also be taken from patients by venous or arterial lines. Once collected, blood samples may be analyzed to obtain medically useful information including, for example, chemical composition, hematology, and coagulation.

Blood samples are routinely taken in evacuated collection tubes. One end of a double-ended needle is inserted into a patient's vein. The other end of the needle then punctures a septum covering the open end of the tube so that the vacuum in the tube draws the blood sample through the needle into the tube.

Capillary blood collection is often performed with multiple devices that require multiple manipulations of the parts and products. These manipulations can lead to inappropriate collection, clotted samples, and/or mislabeling of a specimen.
US 4,920,977 A describes a blood collection assembly with lancet assembly and micro collection tube. In operation a body of the lancet assembly is moved based on contact of a front end of the body with a patient's skin which exposes a blade based on movement of the body that compresses a spring and allows the blade to puncture the patient's skin.
WO 2015/020609 A1 related to finger-pricking devices for extracting capillary whole blood for in-vitro diagnostic testing. WO 2015/020609 discloses a blood collection means that is a measurement and collection tube. The incorporated blood measurement and collection tube enables a user single-handedly to prick his finger with safety lancet, milk the lancet site to obtain a desirable size of blood droplet, and collect and measure at the same time the desired volume of blood.

### SUMMARY OF THE DISCLOSURE

The present disclosure provides a biological fluid collection device that includes a first end, a second end, and a sidewall extending therebetween defining a tube body. The biological fluid collection device further includes a sample collection portion having a cavity defined by a portion of the sidewall adjacent the first end and a puncturing element adjacent the second end.
The cavity has a sample collection bottom surface and an external diameter. The puncturing element is adapted for movement between a pre-actuated position, in which the puncturing element is retained within the biological fluid collection device and a puncturing position, in which at least a portion of the puncturing element extends through the second end of the tube body. A portion of the tube body surrounds the puncturing element in the puncturing position. The portion of the sidewall surrounding the puncturing element has an external diameter that is substantially the same as the external diameter of the sample collection portion.

Advantageously, a biological fluid collection device of the present disclosure can be used to both pierce a patient's skin surface to initiate blood flow, as shown in FIGS. 4 and 5, and to collect a blood sample within the cavity of the tube, as shown in FIG. 7. The cavity is adapted to receive a blood sample therein.

In one configuration, the biological fluid collection device further includes a middle portion such that the sample collection portion is disposed between the first end and the middle portion and the puncturing element is disposed between the middle portion and the second end. The portion of the sidewall defining the sample collection portion may be co-extensive with the portion of the sidewall surrounding the puncturing element. The sample collection portion and the puncturing element may also be integral to the biological fluid collection device.

In one example, the puncturing element is part of a contact activated lancet device. The contact activated lancet device may include a lancet shield having a rearward end and a forward end. The rearward end has a retaining hub for restraining the lancet shield with respect to the biological fluid device while the forward end extends through the second end of the tube body and defines an opening through which the puncturing element extends in the puncturing position. The lancet safety shield includes an enlarged portion disposed at the forward end of the lancet shield for limiting proximal movement of the shield with respect to the tube body. The forward end of the lancet safety shield includes a rounded contact surface. The lancet shield is axially moveable with respect to the tube body.

In one example, the biological fluid collection device further includes a removably attachable cap for sealing the first end of the biological fluid collection device. The cap may be a flip cap. In another example, the biological fluid collection device may include a closure member secured to a portion of the biological fluid collection device to seal the first end thereof.

The biological fluid collection device may further include, a sample stabilizer disposed within the cavity, a label affixed to a portion of the device, and/or a tab member removably securable to the biological fluid collection device to enclose and shield the puncturing element. In one configuration, a part of the sample collection portion of the tube body is resiliently deformable to facilitate expulsion of a sample collected therein.

In one particular example, the biological fluid collection device may have an external diameter of 13 mm and an overall height of 75 mm. Thus, the biological fluid collection device is advantageously the same length and diameter of a standard blood collection tube, and therefore, is compatible with the standard instruments and racks designed to be used with standard blood collection tubes.

The present disclosure also provides a biological fluid collection device having a tubular body having a first end, a second end, and sidewall extending therebetween defining a tubular body. The biological fluid collection device further includes a sample collection portion having a cavity defined by a portion of the tubular body adjacent the first end and a puncturing element disposed at least partially within the tubular body and adjacent the second end. The cavity includes a sample collection bottom surface. The puncturing element is adapted for movement between a pre-actuated position wherein the puncturing element is retained within the biological fluid collection device and a puncturing position wherein at least a portion of the puncturing element extends through the second end of the tubular body. The tubular body has a constant external diameter. The constant external diameter may be 13 mm.

### SUMMARY OF THE INVENTION

In accordance with the present invention, there is provided a biological fluid collection device as defined in claim 1. In addition, further advantageous embodiments follow from the dependent claims.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:

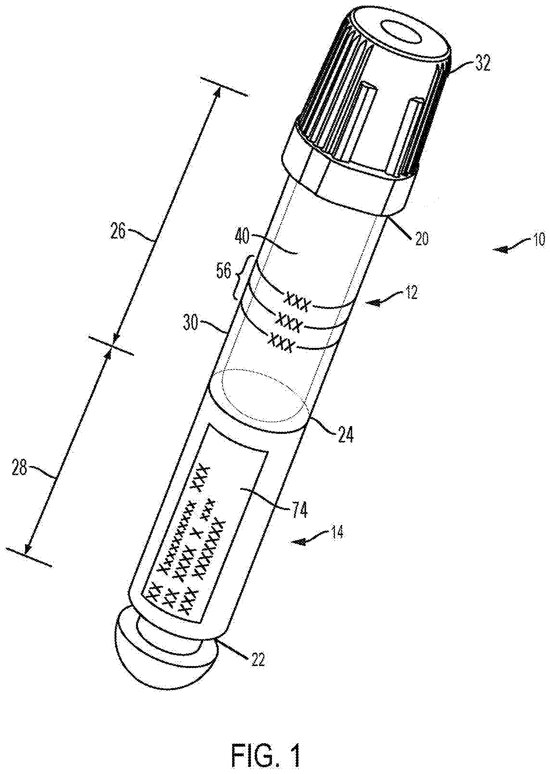

**FIG. 1** is a perspective view of a biological fluid collection device in accordance with an embodiment of the present invention.

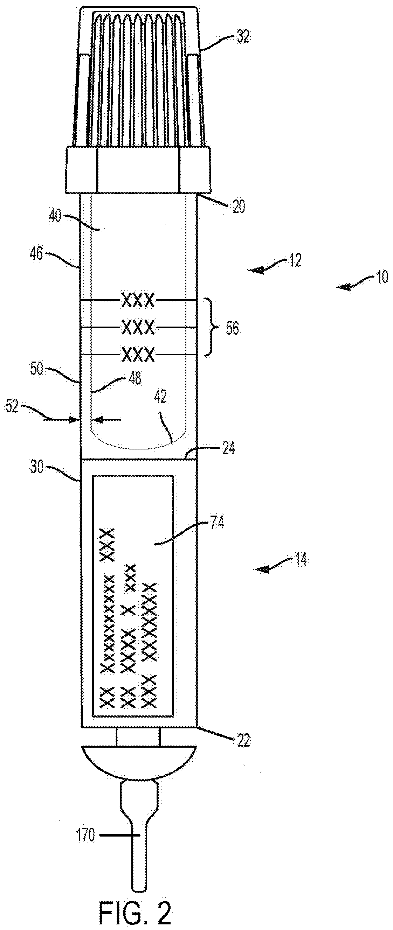

**FIG. 2** is a side plan view of a biological fluid collection device in accordance with an embodiment of the present invention.

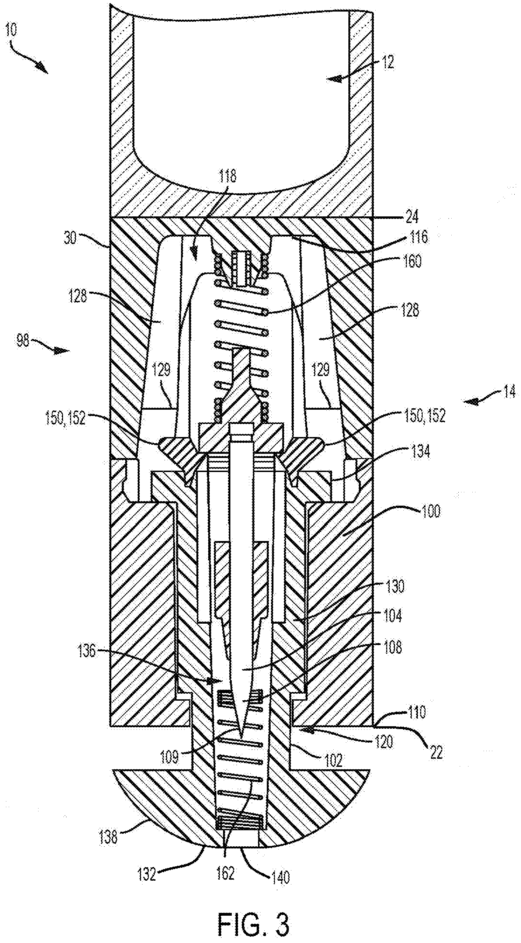

**FIG. 3** is a cross-sectional view of a portion of the biological fluid collection device of FIG. 1 with a puncturing element in a pre-actuated position.

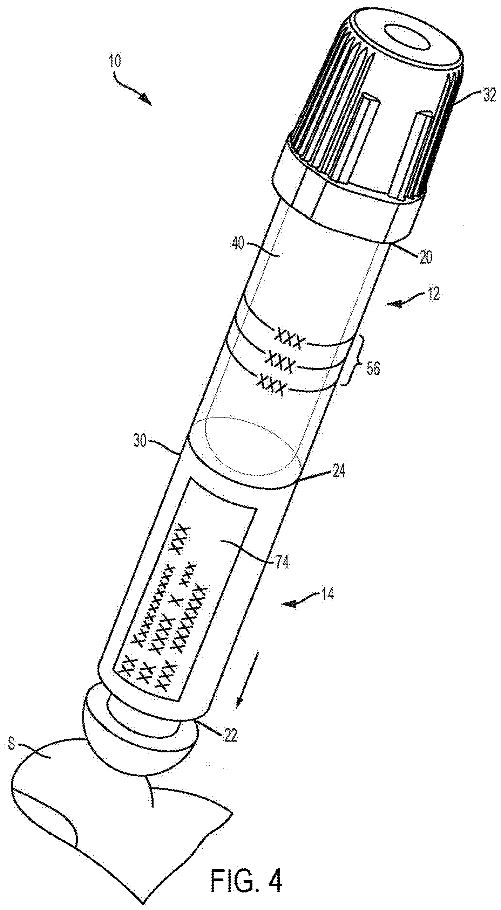

**FIG. 4** is a perspective view of the biological fluid collection device of **FIG.** 1 positioned adjacent a finger of a patient.

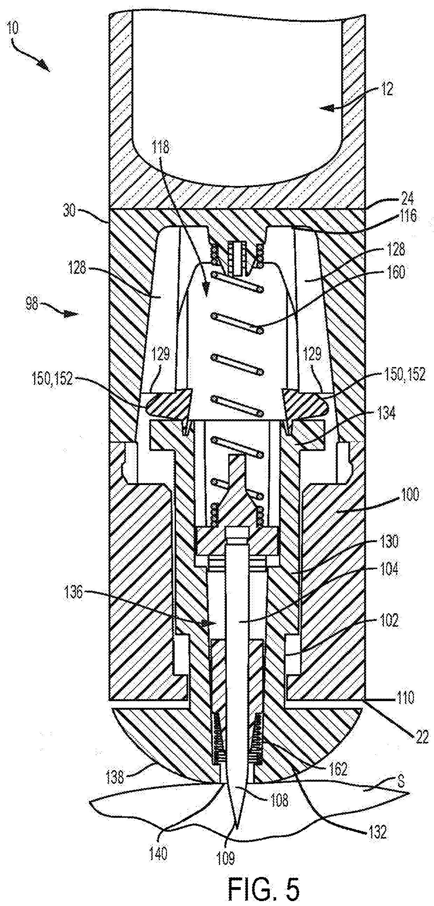

**FIG. 5** is a cross-sectional view of a portion of the biological fluid collection device of FIG. 1 with the puncturing element in a puncturing position.

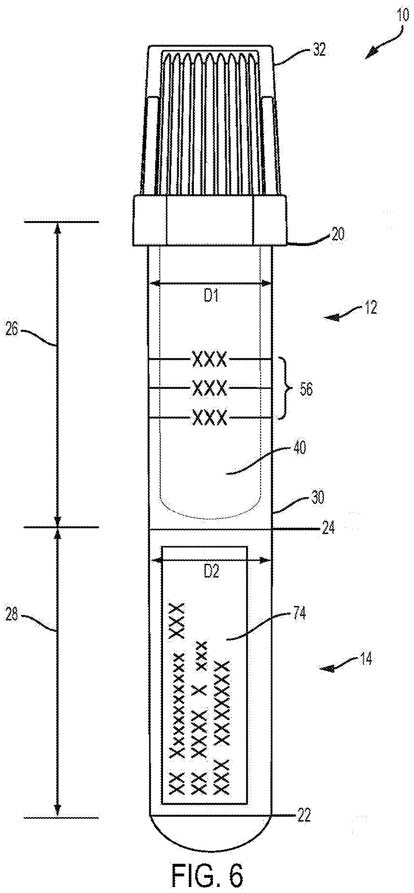

**FIG. 6** is a side plan view of a biological fluid collection device in accordance with an embodiment of the present invention.

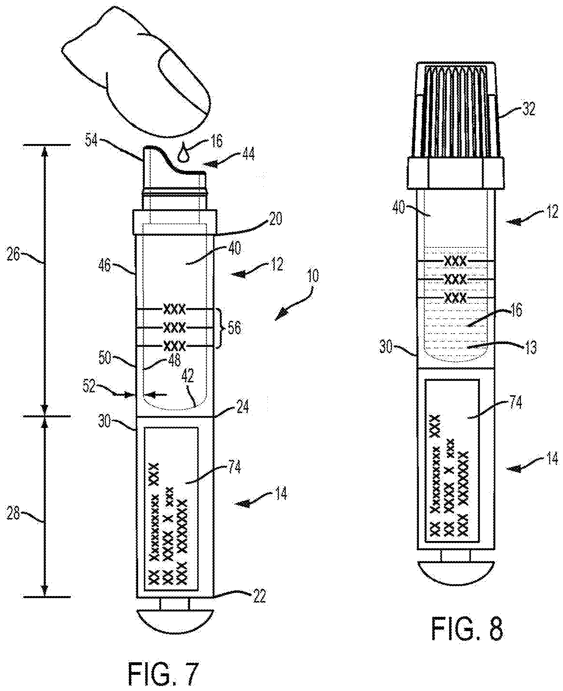

**FIG. 7** is a side plan view of a finger of a patient positioned adjacent a first end of a biological fluid collection device so that a blood sample is collected within a cavity of a sample collection portion of the biological fluid collection device in accordance with an embodiment of the present invention.
**FIG. 8** is a side plan view of the biological fluid collection device of **FIG. 1** with a blood sample received within a cavity of a sample collection portion of the device.

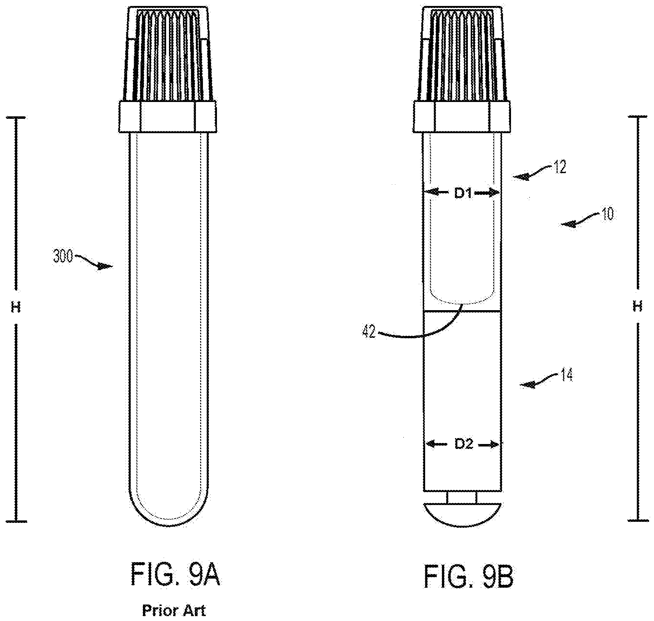

**FIG. 9A** is a side plan view of a standard blood collection tube.
**FIG. 9B** is a side plan view of a biological fluid collection device in accordance with an embodiment of the present invention.

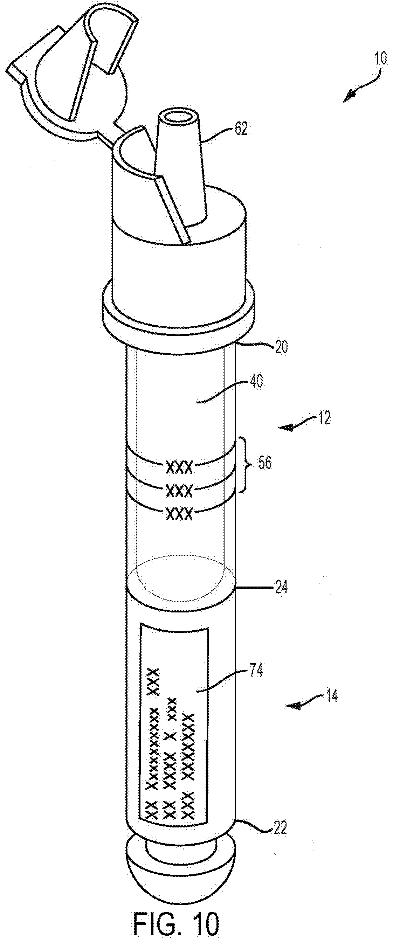

**FIG. 10** is a perspective view of a biological fluid collection device in accordance with another embodiment of the present invention having a flip cap in an open position.

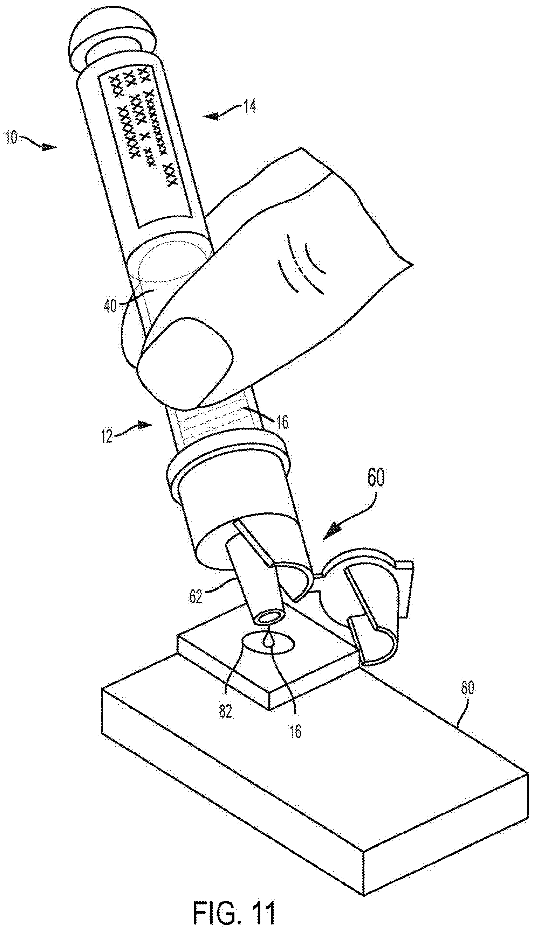

**FIG. 11** is a perspective view of a biological fluid collection device and a point-of-care testing device in accordance with an embodiment of the present invention.

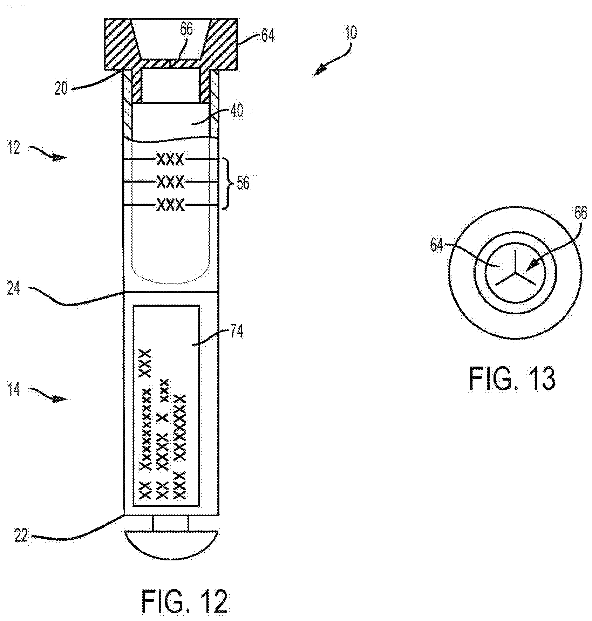

**FIG. 12** is a side plan view of a biological fluid collection device in accordance with another embodiment of the present invention.
**FIG. 13** is a top plan view of the biological fluid collection device of **FIG. 12** having a closure member in a closed configuration.

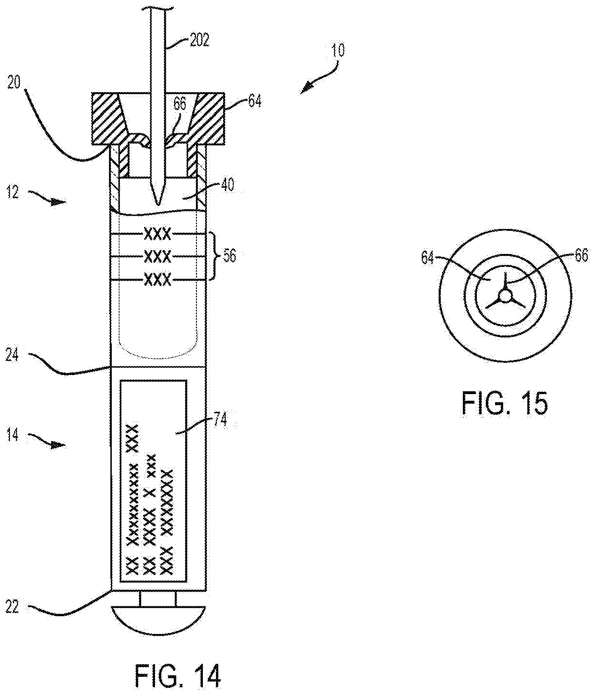

**FIG. 14** is a side plan view of the biological fluid collection device of **FIG. 12****,** with a pipette breaking a portion of the closure member.
**FIG. 15** is a top plan view of the biological fluid collection device of **FIG. 12****,** with a portion of a closure member in an open configuration.

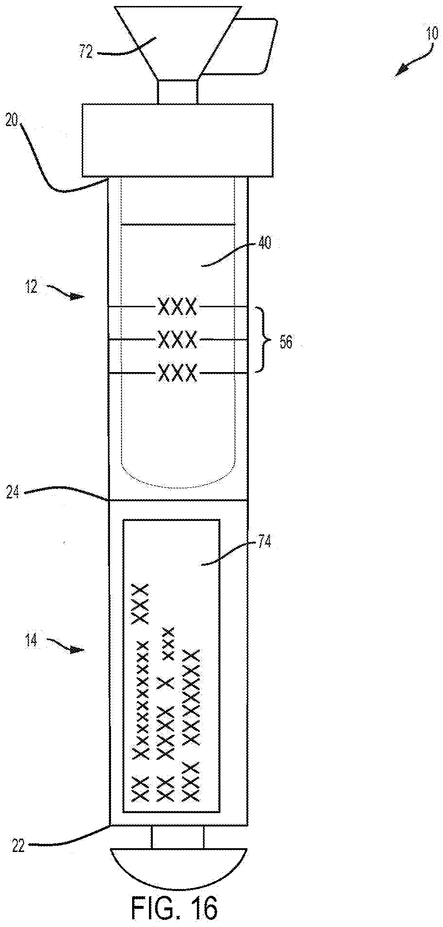

**FIG. 16** is a side plan view a biological fluid collection device in accordance with an embodiment of the present invention having a funnel positioned at a first end of the biological fluid collection device.

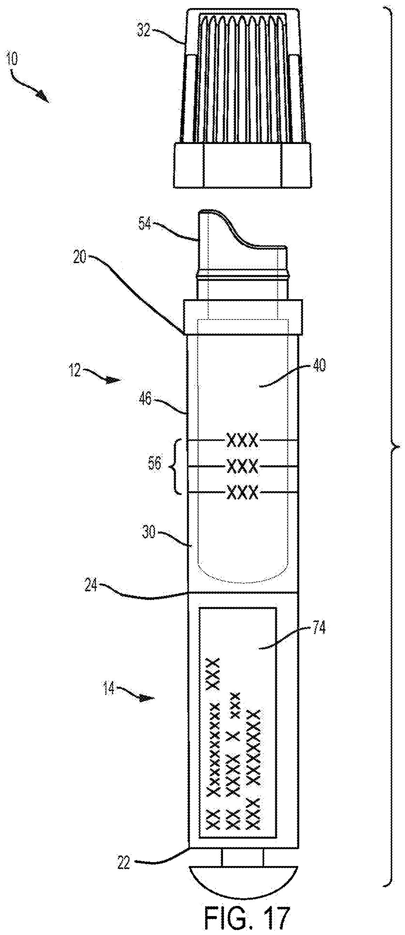

**FIG. 17** is an exploded, side plan view of the biological fluid collection device of **FIG. 1****.**

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the scope of the present invention.

References to "embodiments" throughout the description which are not under the scope of the appended claims represent possible examples and are therefore not part of the present invention unless the context clearly dictates otherwise. The invention is defined by the appended claims.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume alternative variations and step sequences, except where expressly specified to the contrary. It is also to be understood that the specific devices and processes illustrated in the attached drawings, and described in the following specification, are simply exemplary embodiments of the invention. Hence, specific dimensions and other physical characteristics related to the embodiments disclosed herein are not to be considered as limiting.

FIGS. 1-8 illustrate an exemplary embodiment of the present disclosure. Referring to FIGS. 1-8, a biological fluid collection device 10 of the present disclosure includes a sample collection portion 12 and a puncturing element 14. The sample collection portion 12 and the puncturing element 14 are integral to the biological fluid collection device 10. The puncturing element 14 includes a portion that is capable of puncturing a skin surface S (FIGS. 4 and 5) of a patient. The sample collection portion 12 is adapted to receive a blood sample 16 (FIGS. 7 and 8) therein. Advantageously, a biological fluid collection device 10 of the present disclosure can be used to both pierce a patient's skin surface S to initiate blood flow, as shown in FIGS. 4 and 5, and to collect a blood sample 16 within a cavity 40 of the sample collection portion 12, as shown in FIG. 7.

Referring to FIGS. 1-8, the biological fluid collection device 10 includes a first end 20, a second end 22, and a sidewall 30 extending therebetween defining a tubular body. A middle portion 24 is located between the first end 20 and the second end 22, thereby forming a first portion 26 between the first end 20 and the middle portion 24 and a second portion 28 between the second end 22 and the middle portion 24. As used herein, the term middle portion 24 refers to any portion located between the first end 20 and the second end 22 and does not specifically refer to a distance halfway between the first and second ends 20, 22. In the exemplary embodiment illustrated by FIGS. 1-8, the first portion 26 directly abuts the second portion 28. However, it is contemplated that the middle portion could also define an elongated section, thereby separating the first portion 26 from the second portion 28.

Referring to FIGS. 1-8, the sample collection portion 12 is adjacent the first end 20 and defines a cavity 40 therein adapted to receive a blood sample 16. In one embodiment, the sample collection portion 12 is formed by the sidewall 30 of the first portion 26. In another configuration, a tube may be disposed between the middle portion 24 and the first end 20. The terms sample collection portion 12 and tube are used interchangeably hereinafter, irrespective of whether the sample collection portion 12 is defined by the sidewall 30 of the first portion 26 or a separate tube 12 disposed between the first end 20 and the middle portion 24. Referring to FIG. 11, the tube 12 can be formed of a material that is resiliently deformable to facilitate expulsion of a sample as is described in more detail below. In one embodiment, a cap 32 is removably attachable to a portion of the biological fluid collection device 10 to seal the first end 20 thereof. The cap 32 is removably attachable to the first end 20 to seal a fluid, such as a blood sample 16, within the cavity 40 of the tube 12. The cap 32 provides a substantially impermeable enclosure with respect to the biological fluid collection device 10, protects the contents of a blood sample 16 contained within the cavity 40 of the tube 12, prevents leakage of the sample 16, and maintains a sealed, sterilized environment within the cavity 40 of the tube 12.

In one embodiment, the tube 12 of the biological fluid collection device 10 may be a sample collection tube. For example, the tube 12 of the biological fluid collection device 10 may be a sample collection tube, such as a proteomics, molecular diagnostics, chemistry sample tube, blood or other bodily fluid collection tube, microtainer tube, coagulation sample tube, hematology sample tube, or a similar tube.

In some embodiments, the tube 12 may also contain additional additives as required for a particular tube function. For example, the tube 12 may contain a clot inhibiting agent, clotting agents, and/or similar additives. These additives may be provided in particle or liquid form and may be coated onto a portion of the tube 12, sprayed onto a portion of the tube 12, and/or located at the bottom of the tube 12.

In one embodiment, the tube 12 may include a sample stabilizer 13 to produce a stabilized biological sample. The sample stabilizer, can be an anticoagulant, or a substance designed to preserve a specific element within the blood such as, for example, RNA, protein analyte, or other element. In one embodiment, the sample stabilizer is heparin or EDTA. In one embodiment, a plurality of biological fluid collection devices 10 could include different sample stabilizers.

The tube 12 may include a closed bottom end 42, an open top end 44, and a tube sidewall 46 extending therebetween. The tube sidewall 46 may include an inner wall surface 48 and an outer wall surface 50 to define a wall thickness 52 therebetween. Any useful wall thickness 52 may suitably be used. For example, in some embodiments, a non-limiting wall thickness 52 of about 25 mil (or about 0.6 mm) to about 50 mil (or about 1.2 mm) could be used.

In one embodiment, the tube 12 may be formed of a plastic material. In another embodiment, the tube 12 may be formed of a glass material. The tube 12 may be made of one or more than one of the following representative materials: polypropylene, polyethylene terephthalate (PET), polyvinyl chloride, polystyrene, glass, and/or combinations thereof.

The tube 12 can include a single wall or multiple wall configurations. Additionally, the tube 12 may be constructed in any practical size for obtaining an appropriate biological sample. For example, the tube 12 may be of a size similar to conventional volume tubes, or microtainer tubes, as is known in the art. In one embodiment, the tube 12 may be sized such that the biological fluid collection device 10 has an external diameter of 13 mm and an overall height of 75 mm. For example, the tube 12 and the puncturing element 14 can be sized such that the biological fluid collection device 10 is 13 x 75 mm. In this manner, referring to FIGS. 9A and 9B, the biological fluid collection device 10 (FIG. 9B) of the present disclosure is able to have the same length and diameter of a standard 13 x 75 mm blood collection tube 300 (FIG. 9A). Advantageously, the biological fluid collection device 10 of the present disclosure is compatible with the standard instruments, racks, and labels that are designed to be utilized with standard 13 x 75 mm blood collection tubes without the use of extra adapters or the need to aliquot off samples. Furthermore, the biological fluid collection device 10 of the present disclosure can be used to both pierce a patient's skin surface S to initiate blood flow, as shown in FIGS. 4 and 5, and to collect a blood sample within the cavity 40 of the tube 12, as shown in FIG. 7.

Referring to FIGS. 7 and 17, in one embodiment, the tube 12 may include an integrated collector portion 54 at the top end 44 of the tube 12. Referring to FIG. 7, the integrated collector portion 54 provides a curved, ergonomic surface that helps a patient to collect a blood sample 16 within the cavity 40 of the tube 12.

In one embodiment, the tube 12 may include a fill line indicator 56 disposed on a portion of the tube 12, e.g., a portion of the tube sidewall 46. The fill line indicator 56 may include fill lines, such as graduations located on the tube sidewall 46, for providing an indication as to the level or amount of fluid contained within the cavity 40 of the tube 12. Such markings may be provided on the outer wall surface 50, the inner wall surface 48, or integrally formed or otherwise within tube sidewall 46. In other embodiments, alternatively, or in addition thereto, the markings may also provide a description of the contents of the tube 12 or other identifying information such as maximum and/or minimum fill lines.

Referring to FIGS. 1-8, in one embodiment, the puncturing element 14 is adjacent the second end 22 and is at least partially disposed within the biological fluid collection device 10 and adapted for movement between a pre-actuated position wherein the puncturing element 14 is retained within the biological fluid collection device 10 and a puncturing position wherein at least a portion of the puncturing element 14 extends through the second end 22 of the biological fluid collection device 10. The puncturing element 14 can be disposed between the middle portion 24 and the second end 22. In one embodiment, the second portion 28 of the biological fluid collection device 10 includes the puncturing element 14.

In one embodiment, the puncturing element 14 is part of a contact activated lancet device 98. In other embodiments, the puncturing element 14 may be part of any type of lancet device.

Referring to FIGS. 3 and 5, in one embodiment, lancet device 98 is disposed between the middle portion 24 and the second end 22 such that the sidewall 30 defines a housing 100. The lancet device generally includes a shield 102 movably associated with the housing 100 and a lancet structure 104 disposed therein. As will be discussed below, the shield 102 is coaxially and movably associated with the housing 100, and is partially disposed within the housing 100, extending partially outward from the housing 100, with the lancet structure 104 contained within and axially or longitudinally movable through the shield 102. The lancet structure 104 includes a puncturing element 14, the lancet structure 104 at least partially disposed within the housing 100 and adapted for movement between a pre-actuated position (FIG. 3) wherein the puncturing element 14 is retained within the housing 100 and a puncturing position (FIG. 5) wherein at least a portion of the puncturing element 14 extends through a forward end 110 of the housing 100.

The housing 100 of the lancet device 98 and the tube 12 may be integral components that form the biological fluid collection device 10. The housing 100 is generally open defining an internal cavity 118, the internal cavity 118 is closed at a rearward end 116 and includes an opening 120 through a forward end 110, through which the shield 102 extends. The sidewall 30 may be an integrally formed structure molded as a single component, or alternatively, may comprise at least two separate elements that are affixed to each other to aid in assembly of the lancet device 98. For example, the at least two separate elements may be affixed together through an appropriate adhesive, or may include an inter-engaging structure providing a mechanical attachment therebetween, such as a frictional fit or a snap fit construction.

In some embodiments, a portion of the biological fluid collection device 10 may include a surface or surfaces for accommodating a user's fingers, such as finger grip indentations, which may be formed as a concave depression or recess. The surfaces may provide ergonomically shaped surfaces that substantially conform to a user's fingertips to aid the user in manipulating the biological fluid collection device 10 and using the lancet device 98 in a blood collection procedure. In some embodiments, a portion of the biological fluid collection device 10 may include structure to generally improve the grip between the biological fluid collection device 10 and the user's fingertips, such as a plurality of longitudinal ribs and troughs extending along the biological fluid collection device 10 and integrally formed with the biological fluid collection device 10, which may provide a visual and tactile cue to the user to instruct the user where to place his or her fingertips.

The shield 102 extends outward from the opening 120 through the forward end 110 of the housing 100. As shown in FIGS. 3 and 5, the shield 102 is a generally cylindrical hollow structure having a shield body 130 extending between a forward end 132 and a rearward end 134, and defining an internal cavity 136 extending therethrough. The forward end 132 of the shield body 130 defines a rounded, enlarged forward end portion 138 including a forward opening 140 therethrough, through which the puncturing element 14 of the lancet structure 104 extends when the lancet device 98 is actuated by the user. The rounded forward end portion 138 generally defines a small contact area about the opening 140 for contacting the intended area on the user's body, e.g., a skin surface S (FIGS. 4 and 5), which is to be punctured by the puncturing element 14. The shield 102 is axially or longitudinally movable within the housing 100. The enlarged forward end portion 138 includes a rear surface 139 configured to contact the forward end 110 of the housing 100 to limit proximal movement of the shield 102 towards the rearward end 116 of the housing 100. The shield 102 and housing 100 may include corresponding guiding surfaces for guiding the shield 102 through the housing 100.

The lancet device 98 further includes a lancet structure 104 disposed within the housing 100, and extending through the shield 102. As shown in FIGS. 3 and 5, the lancet structure 104 includes a puncturing element 14, shown in the form of lancet 108 defining a puncturing end 109 at the forward end thereof. The lancet structure 104 is adapted for axial or longitudinal movement through the internal cavity 136 of the shield body 130 between an initial armed or pre-actuated position with the puncturing end 109 maintained within the shield body 130 (FIG. 3) to a puncturing position in which the puncturing end 109 extends beyond the forward opening 140 of shield body 130 (FIGS. 5). The puncturing end 109 is adapted for puncturing the skin of a patient, and may define a pointed end, a blade edge, and the like. The puncturing end 109 may include a preferred alignment orientation, such as with a pointed end of a blade aligned in a specific orientation.

As shown in FIGS. 3 and 5, a retaining hub 150 is provided at the rearward end 134 of the shield body 130. The retaining hub 150 is provided as a separate structure disposed or retained within the rearward end 134 of shield body 130. In one embodiment, the shield body 130 may include a surface for supporting and positioning the retaining hub 150 to assist in assembly. In another embodiment, the retaining hub 150 may be molded or formed directly onto the shield body 130.

The retaining hub 150 defines a lever structure or lever element 152 for retaining the lancet structure 104 in an initial armed position retracted within the housing 100 as shown in FIG. 3. The retaining hub 150 and lancet structure 104 are in interference engagement with each other, such that the retaining hub 150 retains the lancet structure 104 in an initial armed position retracted within housing 100.

Moreover, the lever element 152 is adapted for engagement with a structure defined within the housing 100. For example, the rearward end 116 of housing 100 may include a structure extending therein, such as an internal contact 128 integrally formed and extending on at least one, and desirably on two opposing inner sidewalls thereof as shown in FIGS. 3 and 5. Each internal contact 128 includes an engagement surface 129 for contacting engagement with a contact surface of the lever element 152, forming a cam surface. In this manner, the pair of internal contacts 128 may engage the lever elements 152, thereby providing a continual cam-like contact surface during pivotal movement of lever element 152.

Movement of the lancet structure 104 through the lancet device 98 is achieved through a biasing force provided through a drive spring 160. The drive spring 160 is adapted to exert a biasing force against the lancet structure 104 to drive the lancet structure 104 through the device toward the puncturing position, and may be disposed between the rearward end 116 of the housing 100 and the lancet structure 104. When the lancet structure 104 is in an armed position, the drive spring 160 exerts a force against the lancet structure 104, such as between the rearward end 116 of housing 100 and the lancet structure 104, biasing the lancet structure 104 toward the puncturing position.

Referring to FIGS. 3 and 5, a retraction spring 162 may be provided at the forward end of the lancet device 98, for retracting the lancet structure 104 within the shield body 130 after the lancet structure 104 is axially moved to the puncturing position. The retraction spring 162 extends between a forward surface of the lancet structure 104 and an inner surface within the rounded forward end portion 138 of the shield body 130. The retraction spring 162 is typically a compression spring, capable of storing energy when in a compressed state.

Referring to FIG. 2, in one embodiment, the lancet device 98 may further include a protective cover or tab member 170 that is removably securable to a portion of the puncturing element 14 to enclose and shield the puncturing element 14 and for protectively covering the lancet device 98 prior to use thereof. In one embodiment, the lancet device 98 may be molded with an overmolded safety feature, e.g., the tab member 170, which covers the puncturing element 14. The tab member 170 can be removed by twisting it off.

The respective elements of the lancet device of the present invention are all typically formed of molded plastic material, such as a medical grade plastic material. The lancet 108 may be constructed of any suitable material adapted for puncturing the skin, and is typically a surgical grade metal such as stainless steel. As described above, the lancet device 98 and the tube 12 are integral components that form the biological fluid collection device 10. In one embodiment, the housing 100 of the lancet device 98 and the tube 12 are formed of the same material and form a sidewall 30 of the biological fluid collection device 10 that houses the cavity 40 of the tube 12 and the lancet device 98.

Referring to FIGS. 1-5, use of the lancet device 98 will now be described. To prepare the lancet assembly for use, the user removes the tab member 170 from the forward end 22 of the biological fluid collection device 10, as shown in FIG. 2. The rounded forward end portion 138 of the shield body 130 may then be contacted with a location on the user's body or another person's body where it is desired to initiate blood flow, such as the patient's skin surface S as shown in FIG. 4. The lancet device 98 of the present disclosure fires and retracts all in a single action.

Once placed against the body, the user exerts a downwardly directed force on the biological fluid collection device 10 forcing the shield body 130 against skin surface S. Since the retaining hub 150 is adjacent to the rearward end 134 of shield body 130, such displacement of the shield body 130 toward the rearward end 116 causes corresponding rearward movement of retaining hub 150 toward the rearward end 116. Such movement causes the drive spring 160 to compress. This compressing of the drive spring 160 arms the drive spring 160 with a biasing force sufficient to propel the lancet structure 104 axially forward through the shield body 130 to the puncturing position, thereby transitioning the lancet structure 104 to an armed position. At this point, however, the lancet structure 104 is still maintained such that the puncturing end 109 is retracted within the shield body 130 due to the interference engagement between the retaining hub 150 and the lancet structure 104.

Such rearward movement of the retaining hub 150 causes the cam surfaces of engagement surfaces 129 of the internal contacts 128 within the rearward end 116 to engage and co-act with the corresponding contact surfaces of the lever elements 152. Accordingly, the corresponding camming contact surfaces provide an actuator element for the lancet device 98. Such engagement and co-action causes the lever elements 152 to pivot to release the lancet structure 104 through the shield body 130. Eventually, such pivoting causes the lever elements 152 to be pivoted to a point at which the interference engagement between the retaining hub 150 and the lancet structure 104 is released, as shown in FIG. 5. The biasing force of the drive spring 160 propels the lancet structure 104 downward away from the rearward end 116 axially through the housing 100 and shield body 130.

After the lancet device 98 of the biological fluid collection device 10 is used to initiate blood flow from a patient's skin surface S, a first drop of blood can be wiped off. Next, a user can remove the cap 32 from the first end 20 of the biological fluid collection device 10, and a blood sample 16 can be collected within the cavity 40 of the tube 12. For example, referring to FIG. 7, in one embodiment, the integrated collector portion 54 provides a curved, ergonomic surface that helps a patient to collect a blood sample 16 within the cavity 40 of the tube 12. In one embodiment, the tube 12 of the biological fluid collection device 10 allows for up to 800 microliters of blood to be collected.

In some embodiments, the tube 12 may contain additional additives as required for a particular tube function. For example, in one embodiment, the tube 12 may include a sample stabilizer to produce a stabilized biological sample. The sample stabilizer, can be an anticoagulant, or a substance designed to preserve a specific element within the blood such as, for example, RNA, protein analyte, or other element. In one embodiment, the sample stabilizer is heparin or EDTA. Such a sample stabilizer may be provided in particle or liquid form and may be coated onto a portion of the tube 12, sprayed onto a portion of the tube 12, and/or located at the bottom of the tube 12.

Once the blood sample 16 is collected within the cavity 40 of the tube 12, the cap 32 can be secured back on the first end 20 of the biological fluid collection device 10 to seal the blood sample 16 within the cavity 40 of the tube 12. Next, the biological fluid collection device 10 can be inverted for mixing the blood sample 16 with a sample stabilizer within the cavity 40 of the tube 12. Thereafter, the biological fluid collection device 10 can be labeled and sent to a lab for processing. Furthermore, the biological fluid collection device 10 may be engaged with a blood testing device or point-of-care testing device 80 as described in detail below.

Referring to FIG. 10, in one embodiment, the biological fluid collection device 10 includes a flip cap 60 that is removably attachable to a portion of the biological fluid collection device 10 to seal the first end 20 thereof.

The flip cap 60 is transitionable between an open position (FIG. 10) and a closed position in which the flip cap 60 seals a fluid, such as a blood sample 16, within the cavity 40 of the tube 12. In the closed position, the flip cap 60 may provide a substantially impermeable and leak proof enclosure with respect to the biological fluid collection device 10, protect the contents of a blood sample 16 contained within the cavity 40 of the tube 12, and maintain a sealed, sterilized environment within the cavity 40 of the tube 12.

Referring to FIGS. 10 and 11, in one embodiment, the biological fluid collection device 10 includes a nozzle 62. In one embodiment, referring to FIG. 11, the nozzle 62 of the biological fluid collection device 10 may be engaged with a blood testing device or point-of-care testing device 80. For example, the nozzle 62 may be placed over a receiving port 82 of the point-of-care testing device 80 as shown in FIG. 11. Next, a clinician may transfer a portion of a blood sample 16 that is contained within the cavity 40 of the tube 12 to the point-of-care testing device 80 in a closed manner, reducing exposure to the clinician and the patient. The tube 12 can be formed of a material that is resiliently deformable. In this manner, to transfer a portion of a blood sample 16 from the biological fluid collection device 10 to a point-of-care testing device 80, a user can squeeze the tube 12 to expel the blood sample 16 as shown in FIG. 11.

The point-of-care testing device 80 is adapted to receive the nozzle 62 of the biological fluid collection device 10 for closed transfer of a portion of the blood sample 16 from the biological fluid collection device 10 to the point-of-care testing device 80. The point-of-care testing device 80 is adapted to receive the blood sample 16 to analyze the blood sample 16 and obtain test results.

Referring to FIGS. 1, 2, 6-8, 10-12, and 17, in one embodiment, the biological fluid collection device 10 includes a label 74 that is capable of being affixed to a portion of the biological fluid collection device 10. In one embodiment, the label 74 is affixed to a portion of the biological fluid collection device 10 that is adjacent the second end 22. For example, the label 74 can be affixed to the second portion 28 of the biological fluid collection device 10.

The label 74 may include any information identifying characteristics of the sample and/or the biological fluid collection device 10 that may be useful to the healthcare practitioner. For example, the information may identify the integrity of the sample, whether the sample has been subjected to appropriate procedures, or whether the appropriate amount of sample has been collected.

Furthermore, the information may relate to maximum temperatures that the container and/or sample may be exposed to, the timeframe that the container and/or sample may be used upon manufacture, shipment, or some other event, or the amount of time that has transpired after collection of the sample into the container.

The label 74 may include machine readable information, electronically readable information, and/or human readable information.

Referring to FIG. 6, a portion of the biological fluid collection device 10 that is between the first end 20 and the middle portion 24 has a first outer diameter D1, and a portion of the biological fluid collection device 10 that is between the second end 22 and the middle portion 24 has a second outer diameter D2. In one embodiment, the first outer diameter D1 is equal to the second outer diameter D2. In this manner, the biological fluid collection device 10 of the present disclosure includes a tube 12 and a puncturing element 14 that are integral to the biological fluid collection device 10, such that the biological fluid collection device 10 may have the same length and diameter of a standard 13 x 75 mm blood collection tube 300 (FIG. 9A). Thus, the biological fluid collection device 10 of the present disclosure is compatible with the standard instruments and racks that are designed to be utilized with standard 13 x 75 mm blood collection tubes without the use of extra adapters or the need to aliquot off samples.

Referring to FIGS. 12-15, in one embodiment, the biological fluid collection device 10 may include a closure member 64 that is secured to a portion of the biological fluid collection device 10 to seal the first end 20 thereof. In one embodiment, the closure member 64 may be an elastomeric stopper, as is well known in the art of blood collection tubes. In some embodiments, the closure member 64 is a puncturable closure. In some embodiments, the closure member 64 includes a frangible portion 66. In one embodiment, the frangible portion 66 includes a three-way slit that can be pierced. The closure member 64 allows for access to the cavity 40 of the tube 12 without having to remove a cap.

After using the lancet device 98 of the biological fluid collection device 10, as described in detail above, to obtain a blood sample 16, a pipette 202 can be used to break the frangible portion 66 of the closure member 64 as shown in FIGS. 14 and 15. Next, a blood sample can be collected within the cavity 40 of the tube 12.

Referring to FIG. 16, in one embodiment, the biological fluid collection device 10 may also include a funnel 72 that helps a user collect a blood sample 16 within the cavity 40 of the tube 12. The funnel 72 is removably attachable to a portion of the first end 20 of the biological fluid collection device 10. Referring to FIG. 16, with the funnel 72 attached to the first end 20 of the biological fluid collection device 10, the funnel 72 is in fluid communication with the cavity 40 of the tube 12. The funnel 72 provides a component that helps a user to collect a blood sample within the cavity 40 of the tube 12. In one embodiment, the funnel 72 can be used to break the frangible portion 66 of the closure member 64 as shown in FIGS. 14 and 15.

While this disclosure has been described as having exemplary designs, the present disclosure can be further modified within the scope of this disclosure. This application is therefore intended to cover any variations, uses, or adaptations of the disclosure using its general principles. Further, this application is intended to cover such departures from the present disclosure as come within known or customary practice in the art to which this disclosure pertains and which fall within the limits of the appended claims.

## Claims

1. A biological fluid collection device (10) having a first end (20), a second end (22), and a sidewall (30) extending therebetween defining a tube body, the biological fluid collection device (10) further comprising:
a sample collection portion (12) having a cavity (40) defined by a portion of the sidewall (30) adjacent the first end (22), the cavity (40) having a sample collection bottom surface and the sample collection portion having an external diameter; and
a puncturing element (14) adjacent the second end (22), the puncturing element (14) adapted for movement between a pre-actuated position, wherein the puncturing element (14) is retained within the biological fluid collection device (10), and a puncturing position, wherein at least a portion of the puncturing element (14) extends through the second end (22) of the tube body;
a lancet structure (104) and a lancet shield (102), wherein the lancet structure (104) comprises the puncturing element (14), wherein the lancet shield (102) has a shield body (130), and wherein the lancet structure (104) is adapted for longitudinal movement through an internal cavity of the shield body (130) between the pre-actuated position in which a puncturing end of the lancet structure (104) is maintained within the shield body (130) and the puncturing position in which the puncturing end (109) extends beyond a forward opening of the shield body (140);
a drive spring (160), wherein the drive spring (160) is adapted to exert a biasing force against the lancet structure (104) to drive the lancet structure (104) toward the puncturing position;
a retraction spring (162) at a forward end of the lancet structure (104), wherein the retraction spring (162) is configured to retract the lancet structure (104) within the shield body (140) after the lancet structure (104) is axially moved to the puncturing position;
wherein a portion of the sidewall (30) surrounds the puncturing element in the puncturing position, and wherein the portion of the sidewall (30) surrounding the puncturing element (14) has an external diameter that is the same as the external diameter of the sample collection portion,
wherein the lancet shield (102) comprises an enlarged portion (138) at the forward end (132) of the lancet shield (102) for limiting proximal movement of the lancet shield (102) with respect to the tube body.

2. The biological fluid collection device (10) of claim 1, wherein the cavity (40) of the sample collection portion (12) is adapted to receive a blood sample therein.

3. The biological fluid collection device of claim 1, further including a middle portion (24), wherein the sample collection portion (12) is disposed between the first end and the middle portion (24), and the puncturing element (14) is disposed between the middle portion (24) and the second end (22).

4. The biological fluid collection device (10) of claim 1, wherein the portion of the sidewall (30) defining the sample collection portion (12) is co-extensive with the portion of the sidewall (30) surrounding the puncturing element (14).

5. The biological fluid collection device (10) of claim 1, wherein the sample collection portion (12) and the puncturing element (14) are integral to the biological fluid collection device (10).

6. The biological fluid collection device (10) of claim 1, wherein the puncturing element (14) is part of a contact activated lancet device (98); and
wherein the contact activated lancet device (98) further comprises a lancet shield (102) having a rearward end (134) and a forward end (132), the rearward end (134) comprising a retaining hub (150) for restraining the lancet shield (102) with respect to the tube body, the forward end (132) extending through the second end (22) of the tube body and defining the forward opening (140) through which the puncturing element (14) extends when the puncturing element (14) is in the puncturing position.

7. The biological fluid collection device (10) of claim 6, wherein the lancet shield (102) is axially moveable with respect to the tube body and/or wherein the forward end of the lancet shield comprises a rounded contact surface.

8. The biological fluid collection device (10) of claim 1, further comprising a removably attachable cap (32) for sealing the first end (22) of the biological fluid collection device, wherein the cap (32) comprises a flip cap.

9. The biological fluid collection device (10) of claim 1, further comprising a label (74) affixed to a portion of the biological fluid collection device (10) and/or further comprising a tab member (170) removably securable to the biological fluid collection device (10) to enclose and shield (102) the puncturing element (14).

10. The biological fluid collection device (10) of claim 1, wherein at least a part of the sample collection portion (12) is resiliently deformable.

11. The biological fluid collection device (10) of claim 1, wherein the biological fluid collection device (10) has an external diameter of 13 mm and an overall height of 75 mm.

12. The biological fluid collection device (10) of claim 2, wherein a sample stabilizer (13) is disposed within the cavity (40) of the sample collection portion.

## Patentansprüche

1. Vorrichtung (10) zum Sammeln von biologischen Fluiden mit einem ersten Ende (20), einem zweiten Ende (22) und einer sich zwischen diesen erstreckenden Seitenwand, die einen Röhrchenkörper bildet, wobei die Vorrichtung (10) zum Sammeln von biologischen Fluiden ferner aufweist:
einen Probensammelbereich (12) mit einem Hohlraum (40), der durch einen Bereich der Seitenwand (30) nahe dem ersten Ende (22) gebildet ist, wobei der Hohlraum (40) eine Probensammelbodenfläche aufweist und der Probensammelbereich einen Außendurchmesser aufweist; und
ein Punktionselement (14) nahe dem zweiten Ende (22), wobei das Punktionselement (14) zum Bewegen zwischen einer vorbetätigten Position, in welcher das Punktionselement (14) in der Vorrichtung (10) zum Sammeln von biologischen Fluiden gehalten ist, und einer Punktionsposition geeignet ist, in welcher mindestens ein Teil des Punktionselements (14) sich durch das zweite Ende (22) des Röhrchenkörpers erstreckt;
eine Lanzettenstruktur (104) und einen Lanzettenschutz (102), bei welcher die Lanzettenstruktur (104) das Punktionselement (14) aufweist, wobei der Lanzettenschutz (102) einen Schutzkörper (130) aufweist, und wobei die Lanzettenstruktur (104) für eine längsgerichtete Bewegung durch einen Innenhohlraum des Schutzkörpers (130) zwischen der vorbetätigten Position, in welcher ein Punktionsende der Lanzettenstruktur (104) in dem Schutzkörper (130) gehalten ist, und der Punktionsposition geeignet ist, in welcher das Punktionsende (109) sich über eine vordere Öffnung des Schutzkörpers (140) hinaus erstreckt;
eine Antriebsfeder (160), wobei die Antriebsfeder (160) geeignet ist, eine Vorspannkraft gegen die Lanzettenstruktur (104) aufzubringen, um die Lanzettenstruktur (105) in Richtung der Punktionsposition zu treiben;
eine Rückzugsfeder (162) an einem vorderen Ende der Lanzettenstruktur (104), wobei die Rückzugsfeder (162) dazu ausgebildet ist, die Lanzettenstruktur ((104) in den Schutzkörper (140) zurückzuziehen, nachdem die Lanzettenstruktur 8104) axial in die Punktionsposition bewegt ist;
wobei ein Bereich der Seitenwand (30) das Punktionselement in der Punktionsposition umgibt, und wobei der Teil der Seitenwand (20), der das Punktionselement (14) umgibt, einen Außendurchmesser aufweist, der gleich dem Außendurchmesser des Probensammelbereichs ist,
wobei der Lanzettenschutz (102) einen erweiterten Bereich (138) an dem vorderen Ende (132) des Lanzettenschutzes (102) aufweist, um die proximale Bewegung des Lanzettenschutzes (102) in Bezug auf den Röhrchenkörper zu begrenzen.

2. Vorrichtung (10) zum Sammeln von biologischen Fluiden nach Anspruch 1, bei welcher der Hohlraum (40) des Probensammelbereichs (12) zum Aufnehmen einer Blutprobe darin geeignet ist.

3. Vorrichtung (10) zum Sammeln von biologischen Fluiden nach Anspruch 1, ferner mit einem Mittelteil (24), wobei der Probensammelbereich (12) zwischen dem ersten Ende und dem Mittelteil (24) angeordnet ist, und das Punktionselement (14) zwischen dem Mittelteil (24) und dem zweiten Ende (23) angeordnet ist.

4. Vorrichtung (10) zum Sammeln von biologischen Fluiden nach Anspruch 1, bei welcher der Teil der Seitenwand (30), der den Probensammelbereich (12) definiert, sich mit dem Teil der Seitenwand (30) erstreckt, der das Punktionselement (14) umgibt.

5. Vorrichtung (10) zum Sammeln von biologischen Fluiden nach Anspruch 1, bei welcher der Probensammelbereich (12) und das Punktionselement (14) mit der Vorrichtung (10) zum Sammeln von biologischen Fluiden einstückig sind.

6. Vorrichtung (10) zum Sammeln von biologischen Fluiden nach Anspruch 1, bei welcher das Punktionselement (14) Teil einer kontakt-aktivierten Lanzettenvorrichtung (98) ist; und
wobei die kontakt-aktivierte Lanzettenvorrichtung (98) ferner einen Lanzettenschutz (102) mit einem hinteren Ende (134) und einem vorderen Ende (132) aufweist, wobei das hintere Ende (134) einen Rückhalteansatz (150) aufweist, um den Lanzettenschutz (102) in Bezug auf den Röhrchenkörper zurückzuhalten, wobei das vordere Ende (132) sich durch das zweite Ende (22) des Röhrchenkörpers erstreckt und die vordere Öffnung (140) bildet, durch welche sich das Punktionselement (14) erstreckt, wenn das Punktionselement (14) sich in der Punktionsposition befindet.

7. Vorrichtung (10) zum Sammeln von biologischen Fluiden nach Anspruch 6, bei welcher der Lanzettenschutz (102) in Bezug auf den Röhrchenkörper axial bewegbar ist und/oder wobei das vordere Ende des Lanzettenschutzes eine abgerundete Kontaktfläche aufweist.

8. Vorrichtung (10) zum Sammeln von biologischen Fluiden nach Anspruch 1, ferner mit einer lösbar anbringbaren Kappe (32) zum Abdichten des ersten Endes (22) der Vorrichtung zum Sammeln von biologischen Fluiden, wobei die Kappe (32) eine Klapp-Kappe aufweist.

9. Vorrichtung (10) zum Sammeln von biologischen Fluiden nach Anspruch 1, ferner mit einem Etikett (74), das an einem Bereich der Vorrichtung (10) zum Sammeln von biologischen Fluiden angebracht ist, und/oder ferner mit einem Ansatzelement (170), das lösbar an der Vorrichtung (10) zum Sammeln von biologischen Fluiden anbringbar ist, um das Punktionselement (14) zu umgeben und abzuschirmen.

10. Vorrichtung (10) zum Sammeln von biologischen Fluiden nach Anspruch 1, bei welcher mindestens ein Teil des Probensammelbereichs (12) elastisch verformbar ist.

11. Vorrichtung (10) zum Sammeln von biologischen Fluiden nach Anspruch 1, bei welcher die Vorrichtung (10) zum Sammeln von biologischen Fluiden einen Außendurchmesser von 13 mm und eine Gesamthöhe von 75 mm aufweist.

12. Vorrichtung (10) zum Sammeln von biologischen Fluiden nach Anspruch 2, bei welcher ein Probenstabilisator (13) in dem Hohlraum (40) des Probensammelbereichs angeordnet ist.

## Revendications

1. Dispositif de collecte de fluide biologique (10) ayant une première extrémité (20), une deuxième extrémité (22) et une paroi latérale (30) s'étendant entre elles définissant un corps de tube, le dispositif de collecte de fluide biologique (10) comprenant en outre :
une partie de collecte d'échantillon (12) ayant une cavité (40) définie par une partie de la paroi latérale (30) adjacente à la première extrémité (22), la cavité (40) ayant une surface inférieure de collecte d'échantillon et la partie de collecte d'échantillon ayant un diamètre extérieur ; et
un élément de perforation (14) adjacent à la deuxième extrémité (22), l'élément de perforation (14) étant adapté pour se déplacer entre une position pré-actionnée, dans laquelle l'élément de perforation (14) est retenu à l'intérieur du dispositif de collecte de fluide biologique (10), et une position de perforation, dans laquelle au moins une partie de l'élément de perforation (14) s'étend à travers la deuxième extrémité (22) du corps de tube ;
une structure de lancette (104) et une protection de lancette (102), où la structure de lancette (104) comprend l'élément de perforation (14), où la protection de lancette (102) a un corps de protection (130), et où la structure de lancette (104) est adaptée pour se déplacer longitudinalement à travers une cavité interne du corps de protection (130) entre la position pré-actionnée dans laquelle une extrémité de perforation de la structure de lancette (104) est maintenue à l'intérieur du corps de protection (130) et la position de perforation dans laquelle l'extrémité de perforation (109) s'étend au-delà d'une ouverture avant du corps de protection (140) ;
un ressort d'entraînement (160), où le ressort d'entraînement (160) est adapté pour exercer une force de sollicitation contre la structure de lancette (104) pour entraîner la structure de lancette (104) vers la position de perforation ;
un ressort de rappel (162) à une extrémité avant de la structure de lancette (104), où le ressort de rappel (162) est configuré pour rétracter la structure de lancette (104) à l'intérieur du corps de protection (140) après que la structure de lancette (104) est déplacée axialement vers la position de perforation ;
où une partie de la paroi latérale (30) entoure l'élément de perforation dans la position de perforation, et où la partie de la paroi latérale (30) entourant l'élément de perforation (14) a un diamètre extérieur qui est identique au diamètre extérieur de la partie de collecte d'échantillon,
où la protection de lancette (102) comprend une partie élargie (138) à l'extrémité avant (132) de la protection de lancette (102) pour limiter le mouvement proximal de la protection de lancette (102) par rapport au corps de tube.

2. Dispositif de collecte de fluide biologique (10) de la revendication 1, dans lequel la cavité (40) de la partie de collecte d'échantillon (12) est adaptée pour recevoir un échantillon de sang dans celle-ci.

3. Dispositif de collecte de fluide biologique de la revendication 1, comportant en outre une partie intermédiaire (24), où la partie de collecte d'échantillon (12) est disposée entre la première extrémité et la partie intermédiaire (24), et l'élément de perforation (14) est disposé entre la partie intermédiaire (24) et la deuxième extrémité (22).

4. Dispositif de collecte de fluide biologique (10) de la revendication 1, dans lequel la partie de la paroi latérale (30) définissant la partie de collecte d'échantillon (12) est coextensive avec la partie de la paroi latérale (30) entourant l'élément de perforation (14).

5. Dispositif de collecte de fluide biologique (10) de la revendication 1, dans lequel la partie de collecte d'échantillon (12) et l'élément de perforation (14) font partie intégrante du dispositif de collecte de fluide biologique (10).

6. Dispositif de collecte de fluide biologique (10) de la revendication 1, dans lequel l'élément de perforation (14) fait partie d'un dispositif de lancette activé par contact (98) ; et
où le dispositif de lancette activé par contact (98) comprend en outre une protection de lancette (102) ayant une extrémité arrière (134) et une extrémité avant (132), l'extrémité arrière (134) comprenant un raccord de retenue (150) pour retenir la protection de lancette (102) par rapport au corps de tube, l'extrémité avant (132) s'étendant à travers la deuxième extrémité (22) du corps de tube et définissant l'ouverture avant (140) à travers laquelle l'élément de perforation (14) s'étend lorsque l'élément de perforation (14) est en position de perforation.

7. Dispositif de collecte de fluide biologique (10) de la revendication 6, dans lequel la protection de lancette (102) est mobile axialement par rapport au corps de tube et/ou dans lequel l'extrémité avant de la protection de lancette comprend une surface de contact arrondie.

8. Dispositif de collecte de fluide biologique (10) de la revendication 1, comprenant en outre un capuchon pouvant être attaché de manière amovible (32) pour sceller la première extrémité (22) du dispositif de collecte de fluide biologique, dans lequel le capuchon (32) comprend un capuchon rabattable.

9. Dispositif de collecte de fluide biologique (10) de la revendication 1, comprenant en outre une étiquette (74) apposée sur une partie du dispositif de collecte de fluide biologique (10) et/ou comprenant en outre un élément de languette (170) pouvant être fixé de manière amovible au dispositif de collecte de fluide biologique (10) pour enfermer et protéger (102) l'élément de perforation (14).

10. Dispositif de collecte de fluide biologique (10) de la revendication 1, dans lequel au moins une portion de la partie de collecte d'échantillon (12) est élastiquement déformable.

11. Dispositif de collecte de fluide biologique (10) de la revendication 1, dans lequel le dispositif de collecte de fluide biologique (10) a un diamètre extérieur égal à 13 mm et une hauteur totale égale à 75 mm.

12. Dispositif de collecte de fluide biologique (10) de la revendication 2, dans lequel un stabilisateur d'échantillon (13) est disposé à l'intérieur de la cavité (40) de la partie de collecte d'échantillon.
